Europäisches Patentamt

European Patent Office

Office européen des brevets

(19)

(11) Publication number: **0 436 373 B1**

# EUROPEAN PATENT SPECIFICATION

(45) Date of publication of patent specification: **24.08.94**  (51) Int. Cl.5: **A61K 31/19**, A61K 9/20

(21) Application number: **90314164.6**

(22) Date of filing: **21.12.90**

(54) **Process for making tablets containing spray-dried naproxen or naproxen sodium.**

(30) Priority: **22.12.89 US 455109**

(43) Date of publication of application:
**10.07.91 Bulletin 91/28**

(45) Publication of the grant of the patent:
**24.08.94 Bulletin 94/34**

(84) Designated Contracting States:
**AT BE CH DE DK ES FR GB GR IT LI LU NL SE**

(56) References cited:
**EP-A- 0 298 666**
**EP-A- 0 350 701**

(73) Proprietor: **SYNTEX PHARMACEUTICALS IN-TERNATIONAL LIMITED**
**Corner House**
**Church Street**
**Hamilton 5 (BM)**

(72) Inventor: **Kuramoto, Roy**
**27 Alverno Court**
**Redwood City, California 94061 (US)**
Inventor: **Pendleton, Randal Owen**
**460 Point San Bruno Boulevard**
**South San Francisco 94080, CA 94080 (US)**
Inventor: **Chowhan, Zadauddin Tanwir**
**1628 Crow Court**
**Sunnyvale, California 94087 (US)**
Inventor: **Hafezzadeh, Hafez**
**5524 Laplata Circle**
**Boulder, CO 80301 (US)**

(74) Representative: **Armitage, Ian Michael et al**
**MEWBURN ELLIS**
**York House**
**23 Kingsway**
**London WC2B 6HP (GB)**

Rank Xerox (UK) Business Services
(3.10/3.09/3.3.3)

## Description

### FIELD OF THE INVENTION

This invention relates to the preparation of certain tablet formulations which comprises spray drying an aqueous slurry or solution comprising the active drug alone or combined with an aqueous mixture comprising excipient materials to form a powder which may be used for direct compression into tablets.

### BACKGROUND OF THE INVENTION

Pharmaceutically active drugs are not generally administered in their pure form. The drugs are generally mixed with large amounts of excipient material to form dosage units for administration to patients. Tablets are one of the most commonly produced dosage units. Processes for producing tablets are known and have been known for a sufficient period of time to allow for the development of a large number of modifications.

There are three basic methods of forming tablets as follows:

(1) a method of direct compression tableting comprising the two steps of powder-mixing and tableting; (2) a method of dry granulation tableting comprising the steps of preliminarily compressing a mixed powder, crushing, sieving and tableting; and (3) a method of wet granulation tableting which requires complicated steps of powder-mixing, kneading, granulation, drying, sieving, mixing and tableting. The last one is employed in connection with formulations with which direct compression tableting and dry granulation tableting are difficult. The well known non-steroidal anti-inflammatory drugs naproxen and naproxen sodium tablets are generally prepared by a wet granulation technique.

There are a number of publications relating to processes for producing tablets and their formulations as discussed below:

U.S. patent 4,159,345, issued June 26, 1979, discloses excipient materials used to improve the flowability, moldability and compressibility of drug and excipient combinations used for tableting.

U.S. patent 4,159,346, issued June 26, 1979, discloses a formulation which is particularly adapted for the wet granulation tableting method.

U.S. Patent 4,721,709, issued January 26, 1988, discloses pharmaceutical compositions containing hydrophobic practically water-insoluble drugs and processes for the preparation of such compositions.

U.S. Patent 4,802,926, issued February 7, 1989, discloses a process for preparing a spray dried lactose which is used in tablet formulations.

European Patent Application 0,298,666, published January 11, 1989, discloses a spray dried ibuprofen composition suitable for direct compression into tablets comprising a spray dried dispersion in water of ibuprofen, pregelatinized starch, a disintegrant and a wetting agent.

U.S. Patent 3,639,170, issued February 1, 1972, discloses a method of spray drying an aqueous slurry of fine lactose crystals.

U.S. Patent 4,760,094, issued July 26, 1988, discloses a process for spray drying a dispersion of acetaminophen and ethyl cellulose in water having a plasticizer dissolved or suspended therein.

EP 350 701 discloses a pharmaceutical composition in which the active substance, i.a. naproxen, is finely divided and coated. The coating masks the taste of the active substance and protects against its irritating effect on mucous membranes.

### SUMMARY OF THE INVENTION

Naproxen and naproxen sodium tablets are generally prepared by a wet granulation technique. The drug substance in powder form is mixed with the excipients such as a disintegrating agent, a dry binder and a diluent. The powder mixture is then granulated by adding or spraying a binder solution until a wet mass is formed. The wet mass is then dried, milled, blended with the lubricant and compressed into tablets.

The present invention is directed to the manufacture of tablets of naproxen and naproxen sodium which comprises the preparation of naproxen and naproxen sodium powder compositions suitable for direct compression into tablets.

One feature of the present invention is the preparation of an aqueous slurry or solution comprising naproxen or naproxen sodium. The aqueous slurry or solution may be obtained directly from the synthesis of the drug.

The aqueous slurry/solution of naproxen or naproxen sodium may be directly spray dried or it may be combined with an aqueous mixture comprising one or more excipient materials, mixed thoroughly and then

2

spray dried. The spray dried powder comprising the drug alone or the active drug in admixture with one or more excipients may then be mixed with additional dry excipients such as a lubricant before compression into tablets.

One advantage of the present invention is that the tablets may be manufactured in a manner which is more energy efficient especially if the aqueous slurry/solution comprising the active drug is taken directly from the synthesis of the drug.

Another advantage of the present invention is that it eliminates a conventional wet granulation step and its subsequent drying and milling, thereby saving energy, labor and capital equipment expenditures.

Another advantage of the present invention is that the spray dried powder of naproxen and naproxen sodium has particles of uniform size which are spherical, highly porous and possess excellent flowability.

Another advantage of the present invention is that it provides the spray dried powder of naproxen and naproxen sodium which has the drug evenly distributed in the excipient materials and the dry powder is directly compressible.

Another advantage of the present invention is that it provides the possibility of varying parameters of the dried powder such as particle size, porosity, particle density, moisture content and flowability.

Still another advantage of the present invention is that the tablets formed have high contents of the active drug. The low level of excipients required by the present invention makes the weight and size of the tablets well adapted for practical and acceptable patient administration.

Still another advantage is that the spray dried powder is highly compressible because of its porous nature, and the tablets prepared therefrom are also readily dissolvable.

Still another advantage of the present invention is that the tablet formulations are stable under normal storage conditions and stress conditions of temperature and relative humidity.

These and other advantages and features of the present invention will become apparent to those persons skilled in the art upon reading the details of the invention as more fully set forth below.

## DETAILED DESCRIPTION AND PREFERRED EMBODIMENTS

Before the present invention and its specific features are disclosed and described, it is to be understood that the terminology used herein is for the purpose of describing particular embodiments only, and is not intended to be limiting since the scope of the present invention will be limited only by the appended claims.

It must be noted that as used in this specification and the appended claims, the singular forms "a," "an" and "the" include plural referents unless the context clearly dictates otherwise. Thus, for example, reference to "an excipient" includes reference to mixtures of such excipient, reference to "the method of spray drying" includes one or more different methods of spray drying known to those skilled in the art.

The present invention requires the use of an aqueous slurry of naproxen and naproxen sodium. The slurry may be obtained directly from synthesis of the drug according to the methods known to those skilled in the art such as those disclosed in U.S. Patents 4,723,033, 4,605,758 and 4,246,164. If the aqueous slurry is taken directly from the synthesis of the drug, it may be necessary to make some adjustments in the water content prior to spray drying, and the wet cake or slurry obtained from the synthesis is milled using suitable milling equipment to obtain the desired range of the particle size distribution.

The naproxen or naproxen sodium slurry may be the aqueous slurry of the active drug which is then mixed either with the excipients in the solid state or with a concentrated aqueous mixture of the excipients to obtain a uniform distribution of naproxen or naproxen sodium and excipients. The total solid contents of the final combined slurry is usually in the range of about 20 to 70%.

In the slurry of naproxen sodium, part or all of naproxen sodium is in solution due to the greater solubility of the drug in water.

The suitable excipients which may be combined with the active drug in the aqueous slurry include binders, disintegrating agents and pigments.

Typical binders that can be used include polyvinyl resins such as polyvinyl alcohol, polyvinyl pyrrolidone, water soluble derivatives of cellulose such as hydroxypropyl methylcellulose, sodium carboxymethyl-cellulose, water soluble gums such as gum acacia, starches such as pregelatinized starches and gelatin. Combinations of binders can also be used such as the combination of gelatin and polyvinyl pyrrolidone and various combinations of those binders mentioned above.

In the present invention, polyvinylpyrrolidone (povidone), in particular povidone K-90, and hydroxypropyl methylcellulose are preferred.

Povidone USP is 1-ethenyl-2-pyrrolidinone polymers or 1-vinyl-2-pyrrolidinone polymers. Povidone is available in different grades which are generally referred to as K-values wherein K-15 has an average molecular weight of about 10,000, K-30 has an average molecular weight of about 40,000, K-60 has an average molecular weight of about 160,000 and K-90 has an average molecular weight of 360,000. Povidone is soluble in water, up to 60%, freely soluble in many organic solvents, including monohydric (such as ethanol and methanol) and polyhydric alcohols, acid esters, ketones, methylene chloride, chloroform. The viscosity of the solution above 10% concentration depends on the molecular weight. Povidone of USP purity is available commercially from GAF Corporation, 1361 Alps Road, Wayne, N.J. 07470-3687 U.S.A. under the trademark PLASDONE® K.A.

Hydroxypropyl methylcellulose is available in grades containing 16.5 to 30.0% of methoxy (-OCH$_3$) and 4.0 to 32.0% of hydroxypropoxy (-OCH$_2$CHOHCH$_3$) groups, and thus in viscosity and thermal gelation temperatures of solutions of specified concentration. Hydroxypropyl methylcellulose is available commercially from the Dow Chemical Company under the trademark METHOCEL®. Methocel® is available in different grades which are generally referred to as Methocel® E, Methocel® F, Methocel® J and Methocel® K.

In addition to binders the final slurry/solution may also include disintegrating agents such as corn starch, croscarmellose sodium or microcrystalline cellulose.

According to the present invention, the preferred disintegrating agent is croscarmellose sodium.

Croscarmellose sodium is a modified cellulose gum which is an internally crosslinked form of sodium carboxymethyl-cellulose (NaCMC) of USP purity. The cross-linking of NaCMC is achieved without the use of potentially dangerous cross-linking additives. Croscarmellose sodium is a pure, white, free flowing powder, which does not interfere with binding or flow at typical use levels. Croscarmellose sodium is essentially water insoluble, but is highly absorbent and provides excellent disintegration and dissolution properties to tablets when used at levels as low as 5%. Croscarmellose Sodium of USP purity is available commercially from FMC Corporation, 200 Market Street, Philadelphia PA 19103, U.S.A. under the tradename Ac Di Sol®.

The aqueous slurry may also contain pigments such as iron oxide and FD&C dyes and lakes.

To prepare the aqueous slurry for spray drying a slurry of naproxen or naproxen sodium, and a mixture of a binder with disintegrating agent, are combined and if needed purified water is added and stirred to form a feed slurry having about 20 to 70% total solids content by weight.

The amounts of the excipients and their ratios to naproxen or naproxen sodium are based on the effectiveness to produce directly compressible powder. The aqueous slurry/solution of naproxen sodium may also be spray dried without the addition of any excipient materials.

The present invention provides unique powder compositions of naproxen and naproxen sodium which comprise 80% or more preferably 90% or more by weight of the active drug, 1 to 6% by weight of a disintegrating agent and 1 to 6% by weight of a binder. A typical spray dried powder composition of naproxen comprises 90% or more by weight of naproxen, 3% or more by weight of a disintegrating agent and 1% or more by weight of a binder. A spray dried naproxen sodium powder composition may contain only naproxen sodium or it may contain 80% or more preferably 90% or more by weight of naproxen sodium in admixture with 1 to 5% by weight of a disintegrating agent and 2 to 6% by weight of a binder.

The aqueous slurry/solution comprising naproxen or naproxen sodium alone or in admixture with excipient materials is subjected to a controlled form of spray drying to produce the desired powder form. This operation can be carried out by the use of conventional spray drying equipment such as a vertical spray dryer or a fluid spray dryer, utilizing atomizing means in conjunction with a spray drying chamber through which hot drying gas is circulated.

The type of atomizing means employed should be such as is capable of handling relatively concentrated slurries to form coarse atomized droplets, with each droplet being of such size that it contains a plurality of the pharmaceutical drug particles. Both pressure spray and centrifugal atomizing systems may be employed satisfactorily. The particular style and type of atomizer employed should be so selected as to provide atomization of relatively concentrated slurries into droplets of the desired mean diameter and size range. By way of examples, centrifugal atomizers of the type disclosed in U.S. Patent Nos. 3,095,149 and 2,814,527 and a pressure nozzle of the type manufactured by the Spraying Systems Company under the trade name "WhirlJet," may be used.

The drying conditions within the spray drying chamber are controlled in such a manner as to provide the desired moisture content in the final powder product. For a particular piece of equipment, and for a given amount of material being supplied to the atomizer, drying conditions can be adjusted by varying the amount of the drying gas supplied, and by adjusting the inlet gas temperature and/or the temperature of the feed flow being spray dried. In general the gas flow rates and the inlet gas temperature can be adjusted to provide maximum drying capacity, without however causing any heat injury to the product. For example, the inlet gas temperature may range from about 85° to 450°C and is more preferably about 100° to 200°C.

However, due to the rapid evaporation of the feed the product in the dryer will not be exposed to any temperature higher than the wet bulb temperature in the inlet air stream. The nozzle air pressure may vary depending on the size of the spray drying equipment.

The final free moisture content of the spray dried powder can be relatively low, such as about 0.5 percent but is generally about 1.0 to 2 percent for naproxen. For naproxen sodium, the spray dried powder usually contains 4 to 7% moisture which gives optimum compression properties. Such a spray dried powder may be packed in moisture proof containers to prevent absorption of atmospheric moisture. The product can be removed from the containers at a later time to be combined with other additional dry excipients for compression into tablets.

The spray drying generally produces particles ranging in size from approximately 10 $\mu$m to approximately 500 $\mu$m. The major part of the granules are of a size ranging from approximately 80 $\mu$m to approximately 400 $\mu$m.

Because of its granular form, with the granules conforming to spheres, the spray dried product is free-flowing. For many industrial and consumer applications flowability is a desirable characteristic. It facilitates handling of the powder in automatic equipment, as for example where the powder product is measured out and introduced into packages or fed into high-speed tableting equipment.

The bulk density of the powder product is similar to or higher as compared with the bulk density of the fine dried drug particles before processing. Thus in typical instances the bulk density may range from 0.35 to 0.55 grams per milliliter.

The spray dried powder of naproxen or naproxen sodium is then mixed with additional dry excipients before compression into tablets of different label strengths ranging from 100 mg to 1100 mg.

The additional dry ingredients may include a lubricant such as magnesium stearate, stearic acid, sodium stearyl fumarate or a mixture of a lubricant with talc (glidant); a binder such as polyvinyl alcohol, polyvinyl pyrrolidone, water soluble derivatives of cellulose such as hydroxypropyl methylcellulose, sodium carboxymethylcellulose, water soluble gums such as gum acacia, starches such as pregelatinized starches and gelatin or combinations thereof; a disintegrating agent such as corn starch, croscarmellose sodium microcrystalline cellulose and the like.

The tablet formulations of the present invention comprise typically 75% or more preferably 80% or more by weight of naproxen or naproxen sodium, 1 to 8% by weight of a binder, 1 to 18% by weight of a disintegrating agent and 0.1 to 3% by weight of a lubricant.

The preferred tablet formulations for naproxen comprise 90% or more by weight of the active drug, 1 to 4% by weight of a binder, 2 to 6% by weight of a disintegrating agent and 0.1 to 1% by weight of a lubricant.

The tablet formulations for naproxen sodium comprise 75% or more preferably 80% or more by weight of the active drug, 2 to 6% by weight of a binder, 1 to 18% by weight of a disintegrating agent and 0.1 to 3% by weight of a lubricant.

The tablets may be compressed on a high speed rotary tablet press. Compressed tablets could be film coated with an appropriate polymer. The polymer could also serve a specific function, for example, enteric coating or controlled release. Colored uncoated tablets could also be produced by dry blending an appropriate pigment with the spray dried material, such as iron oxide pigments or FD&C dyes.

Due to the low level of the excipients present in the tablet formulations, the tablets prepared according to the present invention have the advantage that their size and weight are well adapted for practical and acceptable patient administration. In addition, the spray dried powder compositions of naproxen and naproxen sodium of the present invention are highly compressible. Utilizing such powder compositions, tablets of acceptable hardness may be manufactured in spite of the low levels of excipient materials. This is particularly surprising for tablet formulations which do not contain substantial amounts of filler materials such as pregelatinized starch, lactose and the like.

The tablets prepared according to the present invention also are readily dissolvable. The high dissolution rate also does not show a significant drop after aging (for example, storage under 93% relative humidity at 40°C). This is due to the intimate mixing of the disintegrating agent with the drug substance and the highly porous nature of the powder compositions. The intimate mixing and high porosity are obtained by the spray drying process of this invention.

EXAMPLES

The following examples are provided so as to provide those of ordinary skill in the art with a complete disclosure and description of how to practice the present invention and are not intended to limit the scope of what the inventors regard as their invention. Efforts have been made to insure accuracy with respect to numbers used (e.g., amounts, temperature, etc.) but some experimental errors and deviation should be accounted for. Unless indicated otherwise, parts are parts by weight, temperature is in degrees C, and pressure is at or near atmospheric.

EXAMPLE 1

An aqueous slurry containing 48.4% solids by weight including 35 kg naproxen, 1.4 kg croscarmellose sodium, 0.7 kg povidone and 37.1 kg of purified water was prepared in a stainless steel container equipped with a suitable stirrer. A fluidized spray dryer was used in which vigorous fluidization of powder in the fluid bed located in the chamber base and recycle of fines from the cyclone attachment resulted in spray drying taking place in a powder laden atmosphere. The slurry was sprayed through a Schlick 2-fluid nozzle with an orifice diameter of 2.0 mm. The nozzle air pressure was maintained at 130 kPa (4 psig), the main inlet air temperature at 239°C, the fluid bed inlet temperature at 88°C and the outlet air temperature at 66°C. The composition of the spray dried material was approximately as follows:

| Naproxen | 94.3% |
|---|---|
| Croscarmellose sodium | 3.77% |
| Povidone K-90 | 1.885% |

The dried product was blended with a lubricant at 0.2% concentration. The mean particle size of the final blend was about 100 to 350 $\mu$m and the final blend had excellent flow through a hopper and was compressible up to 98-177N (10 to 18 Kp) hardness on a rotary tablet press.

Dissolution Test

Dissolution Conditions: U.S.P. Method

0.1 M, pH 7.4 phosphate buffer - Dissolve 2.62 g of monobasic sodium phosphate and 11.50 g of anhydrous dibasic sodium phosphate in 1000 ml of water, and mix.
Medium: 0.1 M, pH 7.4 phosphate buffer; 900 ml.
Apparatus 2: 50 rpm.
Time: 45 minutes.
Procedure - Determine the amount of $C_{14}H_{14}O_3$ dissolved from ultraviolet absorbances at the wavelength of maximum absorbance at about 332 nm of filtered portions of the solution under test, suitably diluted with 0.1 M, pH 7.4 phosphate buffer, in comparison with a standard solution having a known concentration of USP Naproxen RS in the same medium.

Results

The initial dissolution of naproxen tablets 500 mg (label strength) prepared according to the present example is shown in Table 1. Table 1 also shows the dissolution of the tablets after storage for 13 weeks under 93% relative humidity at 40°C. No significant change in the dissolution rate was observed. These results obtained from accelerated conditions also confirm the stability of the tablet formulations under normal storge conditions.

EXAMPLE 2

An aqueous solution containing 34.6% solids by weight which slurry will include 34.6 kg of naproxen sodium and 65.4 kg of purified water was prepared in a stainless steel container equipped with a suitable stirrer at a temperature of 45°C. A fluidized spray dryer was used in which vigorous fluidization of powder in the fluid bed located in the chamber base and recycle of fines from the cyclone attachment resulted in spray drying taking place in a powder laden atmosphere. The slurry was sprayed through a Schlick 2-fluid nozzle with an orifice diameter of 2.0 mm. The nozzle air pressure was maintained at 130 kPa (4 psig), the main inlet air temperature at 179-186°C, the fluid bed inlet temperature at 100-112°C and the outlet air temperature at 61-62°C. The spray dried naproxen sodium powder was thus obtained.
The spray dried naproxen sodium powder (83.5% by weight) was blended with additional dry excipients, microcrystalline cellulose (15% by weight) and magnesium stearate (1.5% by weight). The final blend had excellent flow through a hopper and was compressible up to 98-196N (10-20 Kp) hardness on a rotary tablet press.

The dissolution of naproxen sodium tablets 275 mg (label strength) prepared according to the present example was tested following the USP dissolution method for naproxen sodium tablets. The active drug was completely dissolved within 30 minutes.

TABLE 1

DISSOLUTION RESULTS FROM SPRAY DRIED NAPROXEN 500 mg TABLET BATCHES

| Batch No. | Conditions | Timepoint (Weeks) | % Dissolved ± RSD 5 min | 10 min | 15 min | 30 min |
|---|---|---|---|---|---|---|
| 1 | Initial | 0 | 97.4 ± 2.4 | 99.1 ± 1.4 | 99.2 ± 1.8 | 99.2 ± 1.8 |
|  | 40°C/95% RH | 3 | 83.5 ± 11.8 | 95.5 ± 4.7 | 97.4 ± 2.4 | 98.0 ± 0.2 |
|  | " | 7 | 74.2 ± 16.5 | 93.1 ± 6.8 | 98.5 ± 2.6 | 100.1 ± 1.1 |
|  | " | 13 | 82.5 ± 2.1 | 98.0 ± 1.6 | 99.9 ± 1.6 | 100.4 ± 1.6 |
| 2 | Initial | 0 | 99.0 ± 2.0 | 100.5 ± 1.8 | 100.6 ± 1.8 | 100.6 ± 1.8 |
|  | 40°C/95% RH | 3 | 89.0 ± 10.5 | 99.3 ± 4.1 | 100.7 ± 1.6 | 101.1 ± 0.9 |
|  | " | 7 | 73.0 ± 19.6 | 94.3 ± 8.0 | 99.5 ± 2.5 | 101.1 ± 0.6 |
|  | " | 13 | 77.1 ± 10.5 | 97.3 ± 4.9 | 100.9 ± 2.2 | 102.2 ± 0.7 |

Claims

Claims for the following Contracting States : AT, BE, CH, DE, DK, FR, GB, IT, LI, LU, NL, SE

1. A process for the manufacture of naproxen and naproxen sodium tablets which process comprises spray drying an aqueous slurry/solution of the active drug alone or in admixture with excipient materials, mixing the spray dried powder with additional dry excipients, and compressing the powder mixture into tablets which tablets comprise 75% or more preferably 80% or more by weight of naproxen or naproxen sodium, 1 to 8% by weight of a binder, 1 to 18% by weight of a disintegrating agent and 0.1 to 3% by weight of a lubricant.

7

2. The process according to Claim 1 wherein the tablets comprise 90% or more by weight of naproxen, 1 to 4% by weight of a binder, 2 to 6% by weight of a disintegrating agent and 0.1 to 1% by weight of a lubricant.

3. The process according to Claim 1 wherein the tablets comprise 75% or more preferably 80% or more by weight of naproxen sodium, 2 to 6% by weight of a binder, 1 to 18% by weight of a disintegrating agent and 0.1 to 3% by weight of a lubricant.

4. A tablet formulation which comprises 75% or more preferably 80% or more by weight of naproxen or naproxen sodium spray dried, 1 to 8% by weight of a binder, 1 to 18% by weight of a disintegrating agent and 0.1 to 3% by weight of a lubricant.

5. The tablet formulation according to Claim 4 which comprises 90% or more by weight of naproxen spray dried, 1 to 4% by weight of a binder, 2 to 6% by weight of as disintegrating agent and 0.1 to 1% by weight of a lubricant.

6. The tablet formulation according to Claim 4 which comprises 75% or more preferably 80% or more by weight of naproxen sodium spray dried, 2 to 6% by weight of a binder, 1 to 18% by weight of a disintegrating agent and 0.1 to 3% by weight of a lubricant.

7. The tablet formulation according to Claim 5 wherein 100mg to 1000mg of naproxen is present.

8. The tablet formulation according to Claim 6 wherein 125mg to 1100mg of naproxen sodium is present.

**Claims for the following Contracting States : ES, GR**

1. A process for the manufacture of naproxen and naproxen sodium tablets which process comprises spray drying an aqueous slurry/solution of the active drug alone or in admixture with excipient materials, mixing the spray dried powder with additional dry excipients, and compressing the powder mixture into tablets which tablets comprise 75% or more preferably 80% or more by weight of naproxen or naproxen sodium, 1 to 8% by weight of a binder, 1 to 18% by weight of a disintegrating agent and 0.1 to 3% by weight of a lubricant.

2. The process according to Claim 1 wherein the tablets comprise 90% or more by weight of naproxen, 1 to 4% by weight of a binder, 2 to 6% by weight of a disintegrating agent and 0.1 to 1% by weight of a lubricant.

3. The process according to Claim 1 wherein the tablets comprise 75% or more preferably 80% or more by weight of naproxen sodium, 2 to 6% by weight of a binder, 1 to 18% by weight of a disintegrating agent and 0.1 to 3% by weight of a lubricant.

4. A process according to claim 2 wherein 100 mg to 1000 mg of naproxen is present.

5. A process according to claim 3 wherein 125 mg to 1100 mg of naproxen sodium is present.

**Patentansprüche**
**Patentansprüche für folgende Vertragsstaaten : AT, BE, CH, DE, DK, FR, GB, IT, LI, LU, NL, SE**

1. Verfahren zur Herstellung von Naproxen- und Naproxen-Natrium-Tabletten, welches Verfahren umfaßt die Sprühtrocknung einer wäßrigen Aufschlämmung/Lösung des Wirkstoffes allein oder in Mischung mit Excipienten, Mischen des sprühgetrockneten Pulvers mit zusätzlichen trockenen Excipienten und Pressen des Pulvergemisches zu Tabletten, welche 75 Gew.-% oder mehr, vorzugsweise 80 Gew.-% oder mehr, Naproxen oder Naproxen-Natrium, 1 bis 8 Gew.-% eines Bindemittels, 1 bis 18 Gew.-% eines Desintegriermittels und 0,1 bis 3 Gew.-% eines Gleitmittels umfassen.

2. Verfahren nach Anspruch 1, worin die Tabletten 90 Gew.-% oder mehr Naproxen, 1 bis 4 Gew.-% eines Bindemittels, 2 bis 6 Gew.-% eines Desintegriermittels und 0,1 bis 1 Gew.-% eines Gleitmittels umfassen.

3. Verfahren nach Anspruch 1, worin die Tabletten 75 Gew.-% oder mehr. vorzugsweise 80 Gew.-% oder mehr, Naproxen-Natrium, 2 bis 6 Gew.-% eines Bindemittels, 1 bis 18 Gew.-% eines Desintegriermittels und 0,1 bis 3 Gew.-% eines Gleitmittels umfassen.

4. Tabletten-Formulierung, welche umfaßt 75 Gew.-% oder mehr, vorzugsweise 80 Gew.-% oder mehr, sprühgetrocknetes Naproxen oder Naproxen-Natrium, 1 bis 8 Gew.-% eines Bindemittels, 1 bis 18 Gew.-% eines Desintegriermittels und 0,1 bis 3 Gew.-% eines Gleitmittels.

5. Tabletten-Formulierung nach Anspruch 4, welche umfaßt 90 Gew.-% oder mehr sprühgetrocknetes Naproxen, 1 bis 4 Gew.-% eines Bindemittels, 2 bis 6 Gew.-% eines Desintegriermittels und 0,1 bis 1 Gew.-% eines Gleitmittels.

6. Tabletten-Formulierung nach Anspruch 4, welche umfaßt 75 Gew.-% oder mehr, vorzugsweise 80 Gew.-% oder mehr, sprühgetrocknetes Naproxen-Natrium, 2 bis 6 Gew.-% eines Bindemittels, 1 bis 18 Gew.-% eines Desintegriermittels und 0,1 bis 3 Gew.-% eines Gleitmittels.

7. Tabletten-Formulierung nach Anspruch 5, worin 100 mg bis 1000 mg Naproxen vorhanden sind.

8. Tabletten-Formulierung nach Anspruch 6, worin 125 mg bis 1100 mg Naproxen-Natrium vorhanden sind.

**Patentansprüche für folgende Vertragsstaaten : ES, GR**

1. Verfahren zur Herstellung von Naproxen- und Naproxen-Natrium-Tabletten, welches Verfahren umfaßt die Sprühtrocknung einer wäßrigen Aufschlämmung/Lösung des Wirkstoffes allein oder in Mischung mit Excipienten, Mischen des sprühgetrockneten Pulvers mit zusätzlichen trockenen Excipienten und Pressen des Pulvergemisches zu Tabletten, welche 75 Gew.-% oder mehr, vorzugsweise 80 Gew.-% oder mehr, Naproxen oder Naproxen-Natrium, 1 bis 8 Gew.-% eines Bindemittels, 1 bis 18 Gew.-% eines Desintegriermittels und 0,1 bis 3 Gew.-% eines Gleitmittels umfassen.

2. Verfahren nach Anspruch 1, worin die Tabletten 90 Gew.-% oder mehr Naproxen, 1 bis 4 Gew.-% eines Bindemittels, 2 bis 6 Gew.-% eines Desintegriermittels und 0,1 bis 1 Gew.-% eines Gleitmittels umfassen.

3. Verfahren nach Anspruch 1, worin die Tabletten 75 Gew.-% oder mehr, vorzugsweise 80 Gew.-% oder mehr, Naproxen-Natrium, 2 bis 6 Gew.-% eines Bindemittels, 1 bis 18 Gew.-% eines Desintegriermittels und 0,1 bis 3 Gew.-% eines Gleitmittels umfassen.

4. Verfahren nach Anspruch 2, worin 100 mg bis 1000 mg Naproxen vorhanden sind.

5. Verfahren nach Anspruch 3, worin 125 mg bis 1100 mg Naproxen-Natrium vorhanden sind.

**Revendications**
**Revendications pour les Etats contractants suivants : AT, BE, CH, DE, DK, FR, GB, IT, LI, LU, NL, SE**

1. Procédé de production de comprimés de naproxène et de sel de sodium de naproxène, qui comprend le séchage par pulvérisation d'une suspension/solution aqueuse du médicament actif seul ou en mélange avec des excipients, l'addition à la poudre séchée par pulvérisation d'autres excipients secs et la compression du mélange en poudre en comprimés qui comprennent 75 % ou de préférence 80 % en poids ou davantage de naproxène ou de sel de sodium du naproxène, 1 à 8 % en poids d'un liant, 1 à 18 % en poids d'un agent de désintégration et 0,1 à 3 % en poids d'un lubrifiant.

2. Procédé suivant la revendication 1, dans lequel les comprimés comprennent 90 ou plus de 90 % en poids de naproxène, 1 à 4 % en poids d'un liant, 2 à 6 % en poids d'un agent de désintégration et 0,1 à 1 % en poids d'un lubrifiant.

3. Procédé suivant la revendication 1, dans lequel les comprimés comprennent 75 % ou de préférence 80 % en poids ou davantage de sel de sodium du naproxène, 2 à 6 % en poids d'un liant, 1 à 18 % en poids d'un agent de désintégration et 0,1 à 3 % en poids d'un lubrifiant.

4. Formulation pour comprimés, qui comprend 75 % ou de préférence 80 % en poids ou davantage de naproxène ou de sel de sodium de naproxène séché par pulvérisation, 1 à 8 % en poids d'un liant, 1 à 18 % en poids d'un agent de désintégration et 0,1 à 3 % en poids d'un lubrifiant.

5. Formulation pour comprimés suivant la revendication 4, qui comprend 90 % en poids ou davantage de naproxène séché par pulvérisation, 1 à 4 % en poids d'un liant, 2 à 6 % en poids d'un agent de désintégration et 0,1 à 1 % en poids d'un lubrifiant.

6. Formulation pour comprimés suivant la revendication 4, qui comprend 75 % ou de préférence 80 % en poids ou davantage de sel de sodium du naproxène séché par pulvérisation, 2 & 6 % an poids d'un liant, 1 à 18 % d'un agent de désintégration et 0,1 à 3 % en poids d'un lubrifiant.

7. Formulation pour comprimes suivant la revendication 5, dans laquelle 100 mg à 1000 mg de naproxène sont présents.

8. Formulation pour comprimés suivant la revendication 6, dans laquelle 125 mg à 1100 mg de sel de sodium du naproxène sont présents.

**Revendications pour les Etats contractants suivants : ES, GR**

1. Procédé de production de comprimés de naproxène et de sol de sodium du naproxène, qui comprend le séchage par pulvérisation d'une suspension/solution aqueuse du médicament actif seul ou en mélange avec des excipients, l'addition à la poudre séchée par pulvérisation d'autres excipients secs et la compression du mélange en poudre en comprimés qui comprennent 75 % ou de préférence 80 % en poids ou davantage de naproxène ou de sel de sodium du naproxène, 1 à 8 % en poids d'un liant, 1 à 18 % en poids d'un agent de désintégration et 0,1 à 3 % en poids d'un lubrifiant.

2. Procédé suivant la revendication 1, dans lequel les comprimés comprennent 90 ou plus de 90 % en poids de naproxène, 1 à 4 % en poids d'un liant, 2 à 6 % en poids d'un agent de désintégration et 0,1 à 1 % en poids d'un lubrifiant.

3. Procédé suivant la revendication 1, dans lequel les comprimés comprennent 75 % ou de préférence 80 % en poids ou davantage de sel de sodium du naproxène, 2 à 6 % en poids d'un liant, 1 à 18 % en poids d'un agent de désintégration et 0,1 à 3 % en poids d'un lubrifiant.

4. Procédé suivant la revendication 2, dans lequel est présente une quantité de 100 mg à 1000 mg de naproxène.

5. Procédé suivant la revendication 3, dans lequel est présente une quantité de 125 mg à 1100 mg de sel de sodium du naproxène.